(19) Europäisches Patentamt
European Patent Office
Office européen des brevets

(11) Publication number: 0 416 016 B1

(12) # EUROPEAN PATENT SPECIFICATION

(45) Date of publication of patent specification: **22.03.95**

(51) Int. Cl.[6]: **C07C 19/08**, C07C 17/00, B01J 27/125

(21) Application number: **89906631.0**

(22) Date of filing: **23.05.89**

(86) International application number:
**PCT/US89/02159**

(87) International publication number:
**WO 89/11467 (30.11.89 89/28)**

(54) **GAS-PHASE FLUORINATION PROCESS.**

(30) Priority: **23.05.88 US 197222**

(43) Date of publication of application:
**13.03.91 Bulletin 91/11**

(45) Publication of the grant of the patent:
**22.03.95 Bulletin 95/12**

(84) Designated Contracting States:
**AT BE CH DE FR GB IT LI LU NL SE**

(56) References cited:
**EP-A- 298 662**
**CA-A- 1 196 345**
**US-A- 2 744 147**
**US-A- 3 607 955**
**US-A- 3 803 241**
**EP-A- 0 282 005**
**GB-A- 1 000 083**
**US-A- 3 258 500**
**US-A- 3 793 229**
**US-A- 4 147 733**

(73) Proprietor: **E.I. DU PONT DE NEMOURS AND COMPANY**
**1007 Market Street**
**Wilmington**
**Delaware 19898 (US)**

(72) Inventor: **MANZER, Leo, E.**
**714 Burnley Road**
**Wilmington, DE 19803 (US)**
Inventor: **RAO, V., N., Mallikarjuna**
**1 Georgetown Avenue**
**Wilmington, DE 19809 (US)**

(74) Representative: **Woodcraft, David Charles et al**
**BROOKES & MARTIN**
**High Holborn House**
**52/54 High Holborn**
**London, WC1V 6SE (GB)**

EP 0 416 016 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

An improved process for the manufacture of 1,1,1-trifluorodichloroethane (FC-123) and/or 1,1,1,2-tetrafluorochloroethane (FC-124), more particularly, a gas-phase reaction of a suitable tetrahaloethylene with hydrogen fluoride in the presence of a selected metal in combination with a high fluorine content aluminum-containing compound, the reaction being conducted under controlled conditions whereby the production of pentafluoroethane is minimized.

### BACKGROUND OF THE INVENTION

Canadian Patent No. 1,196,345 (1985) describes a process for the preparation of $CF_3CHXY$ (X = H, F; Y = H, F, Cl, Br, I) by addition of HF to the corresponding ethylene in the presence of chromium oxyfluoride at 20-200 °C, especially 60-180 °C.

U.S. 3,755,477 describes a process for producing fluorinated aliphatic hydrocarbons which comprises fluorinating a halogenated aliphatic hydrocarbon, including tetrachloroethylene and chlorotrifluoroethylene, by reaction in the gas phase with hydrogen fluoride in the presence of a steam-treated and calcined chromium oxide catalyst prepared by a multi-step process. Example 23, col. 5, shows tetrachloroethylene as a raw material with formation of $CF_3CHCl_2$ (20%), $CF_3CHClF$ (20%), $CF_3CHF_2$ (30%), and $CF_3CClF_2$ -(20%) at 10/1 $HF/C_2Cl_4$ mol ratio, 5.4 seconds contact time and 360 °C reaction temperature. Example 24, col. 5, shows chlorotrifluoroethylene as a raw material with formation of $CF_2=CF_2$ (20%) and $CF_3CHClF$ (13%) at 1.8/1 $HF/C_2ClF_3$ mol ratio, 4 seconds contact time and 320 °C reaction temperature. In these examples, less desirable pentafluorinated products are obtained in a greater amount than the desired tri- and tetrafluoro products.

U.S. 3,258,500 describes a process for the catalytic vapor phase reaction of HF with halohydrocarbons, including tetrachloroethylene including chlorotrifluoroethylene, employing a catalyst that consists essentially of a heat-activated anhydrous chromium (III) oxide which may be supported on alumina. This catalyst is highly active. Example 17, col. 14, shows fluorination of tetrachloroethylene with this catalyst, like that of the above '477 patent, produces large quantities of the less desirable highly fluorinated pentafluoroethane. At 400 °C the product distribution is 35.0% pentafluoroethane, 9.2% 1-chloro-1,2,2,2-tetrafluoroethane, and 3.5% 1,1-dichloro-2,2,2-trifluoroethane. At 300 °C the product distribution is 38.3% 1-chloro-1,2,2,2-tetrafluoroethane, 25.4% pentafluoroethane, and 16.0% 1,1-dichloro-2,2,2-trifluoroethane. Example 20, col.15, shows chlorotrifluoroethylene yields $CF_3CHF_2$ as the major product at 400 °C.

GB 1,000,485 describes a process for the preparation of organic fluorinated compounds by fluorination of halo-olefins in the gaseous phase and at a temperature preferably within the range 200 °C to 400 °C. The catalyst consists essentially of partially fluorinated alumina impregnated with one or more polyvalent metal halides. The polyvalent metal may be chromium, cobalt, nickel, or manganese. The total content of polyvalent metal halide expressed as oxide is not more than 15% by weight of the partially fluorinated (70-80%) alumina expressed as alumina. Example 4, (Table 4) shows reaction of tetrachloroethylene with HF over such catalyst yields $CF_3CHC1_2$ as the major product at 220-290 °C. In addition, the patent states that if fluorination of the catalyst is excessive, the activity of the catalyst is impaired (page 3, column 2, lines 85-87).

The references do not disclose how to produce selectively both 1,1,1-trifluorochloroethane and 1,1,1,2-tetrafluorochloroethane while minimizing the production of the pentafluoroethanes, especially at high tetrahaloethylene conversions.

EP 282 005 published 14 September 1988 and designating Switzerland/Liechtenstein, Germany, Spain, France, UK, Netherlands and Sweden describes a process for the preparation of 1,1,1-trifluoro-2,2-dichloroethane which comprises reacting perchloroethylene with hydrogen fluoride in the gas phase in the presence of a catalyst comprising 1 to 15 weight % $Cr_20_3$ calculated as Cr supported on $AlF_3$ in the gamma and/or beta form.

EP 298 562 published 11 January 1989 discloses a process for the preparation of 1,1,1-triflurorodichloroethane and 1,1,1,2-tetrafluorochloroethane by fluorination of a tetrahaloethylene $C_2Cl_{4-x}F_{x1}$ wherein x = 0 to 3, comprising contacting in the gaseous phase at about 300 °C to about 450 °C said tetrahaloethylene and HF with a catalyst comprising at least one metal in an oxidation state greater than zero, said metal selected from the group consisting of chromium, manganese, nickel, and cobalt, on a support comprising aluminium, oxygen, and fluorine in such proportions that the fluorine content corresponds to an $AlF_3$ content of at least 90% by weight of the catalyst composition exclusive of the metal, said $AlF_3$ content being obtained by pretreatment with HF.

The process of the instant invention achieves the desired high degree of selectivity by minimizing the formation of the pentafluoroethane, through catalyst selection and control of the reaction variables as discussed below and illustrated in the Examples.

## SUMMARY OF THE INVENTION

What has been discovered is a process for the preparation of 1,1,1-trifluorodichloroethane and/or 1,1,1,2-tetrafluorochloroethane by fluorination of a tetrahaloethylene, $C_2C1_4\text{-}_xF_x$, wherein x = 0 to 3, comprising contacting in the gaseous phase at 275°C to 450°C said tetrahaloethylene and HF with a catalyst comprising a metal in an oxidation state greater than zero, said metal selected from the group consisting of chromium, manganese, rhodium, nickel, cobalt and mixtures thereof, said metal in combination with an aluminium-containing compound consisting essentially of aluminium and fluorine in such proportions that the fluorine content corresponds to an $AlF_3$ content of at least 90% by weight of the catalyst composition exclusive of the metal, said $AlF_3$ content being obtained by pretreatment of the unfluorinated catalyst with a vaporizable fluorine-containing compound.

## DETAILS OF THE INVENTION

The tetrahaloethylene of this invention is defined by the formula $C_2Cl_{4-x}F_x$, wherein x = 0 to 3, and includes $CCl_2=CCl_2$, $CClF=CCl_2$, $CClF=CClF$, $CF_2=CCl_2$, and $CF_2=CClF$, and mixtures of these. Tetrachloroethylene is preferred.

By a high fluorine content aluminum-containing compound is meant a composition comprising aluminum and fluorine in such proportions that the total fluorine content of the catalyst composition taken as $AlF_3$ corresponds to at least 90 weight percent, exclusive of the metal, i.e., chromium, manganese, rhodium, nickel and cobalt, preferably 95 weight percent $AlF_3$ or more. The remainder of the aluminum-containing compound may include alumina or aluminum oxyfluoride.

The high fluorine content aluminum-containing compound can be prepared in-situ by exhaustive fluorination of alumina impregnated with at least one chromium, manganese, rhodium, nickel or cobalt compound which may be in the form of the oxide, oxyhalide, halide or pseudohalide or such other form which is convertible to the fluoride or oxyfluoride under the conditions of the pretreatment step described herein. The halides include fluorides, chlorides, or bromides. The pseudohalides include cyanides, cyanates and thiocyanates. The preferred metals are manganese, rhodium, nickel and cobalt. The most preferred metal is cobalt.

The total content of chromium, manganese, rhodium, nickel or cobalt expressed as the divalent oxide is not more than 20% by weight of the supported catalyst.

The catalyst of the instant invention can be prepared prior to reaction with the tetrahaloethylene, by impregnating $Al_2O_3$ with the desired metal compound and treatment with a fluorine-containing compound, such as HF, $SF_4$, $CCl_3F$, $CClF_2$, $CHF_3$ or $CCl_2FCClF_2$, at elevated temperatures until the desired degree of fluorination is obtained, e.g., at 200 degrees Centigrade to 450 degrees Centigrade. The treatment with HF or other vaporizable fluorine-containing compound can conveniently be done in the reactor which is to be used for contacting tetrachloroethylene with HF.

By vaporizable fluorine-containing compound is meant a fluorine-containing compound which will convert the unfluorinated catalyst of the instant invention to the desired degree of fluorination using the pretreatment conditions described herein.

A suitable catalyst may be prepared, for example, as follows:

A quantity of $Al_2O_3$ is impregnated with a solution of a catalytically effective amount of one or more metal halides or pseudohalides of chromium, manganese, rhodium, nickel, or cobalt. By catalytically effective amount is meant an amount of the metal expressed as the divalent oxide between 0.02 to 20 weight percent of the alumina support, preferably 0.1 to 5 weight percent. The impregnated $Al_2O_3$ can be dried until essentially all moisture is removed, e.g., for about 18 hours at about 300°C. The dried catalyst is then transferred to the reactor to be used. The temperature is then gradually increased to about 400°C while maintaining a flow of $N_2$ through the reactor to remove any remaining traces of moisture from the catalyst and the reactor. The temperature is then lowered to about 200°C and HF diluted with $N_2$ is passed through the reactor. The $N_2$ can be gradually reduced until only HF is being passed through the reactor. At this point the temperature can be increased to about 450°C and held at that temperature to convert the impregnated $Al_2O_3$ to a fluoride content corresponding to at least 90% $AlF_3$, e.g., for 15 to 300 minutes, depending on the HF flow and the catalyst volume.

The reaction of the tetrahaloethylene with HF in the presence of the catalyst of the instant invention is conducted at 275 °C to 450 °C, preferably 300 °C to 400 °C, and most preferably 325 °C to 350 °C, with a contact time of 5 to 100 seconds, preferably 10 to 90 seconds, and most preferably 15 to 60 seconds.

The molar ratio of HF to the tetrahaloethylene can range from 1/1 to 20/1, preferably 3/1 to 10/1, and most preferably 4/1 to 7/1.

If desired, the process may be run at temperatures less than 275 °C but for a given HF/$C_2Cl_4$ ratio and contact time a decline in temperature results in a decrease in conversion of the $C_2Cl_4$, a decrease in the production of $CF_3CHCl_2$ and $CF_3CHClF$, and an increase in the formation of the underfluorinated products such as $C_2HCl_4F$ and $C_{2Cl3}F_2$. However, if the goal is to manufacture the under fluorinated products, the catalyst of this invention is well suited for this purpose when used at temperatures of less than 275 °C.

In general, with a given catalyst composition, the higher the temperature, the greater the HF/tetrahaloethylene mol ratio, and the longer the contact time, the greater is the conversion of the tetrahaloethylene to fluorinated products. The above variables can be balanced, one against the other, so that formation of FC-123 is favored over FC-124 and the production of these two compounds taken together is maximized and that of higher fluorinated products minimized.

A key feature of the invention is that through catalyst selection and process control, as described herein, the desired tri- and tetrafluoro products can be obtained as the major products at high tetrahaloethylene conversions, normally between 30% and 90%. Preferably, the reaction variables are controlled so as to keep the production of the pentafluoro product below 10 area percent, as determined gas chomatographically, of the products produced. Thus, as illustrated in the examples with tetrachloroethylene, the tri- and tetrafluoro products are obtained in very high yields while minimizing the production of higher fluorinated products even at high conversions of tetrachloroethylene.

Underfluorinated compounds formed during the course of the reaction, such as $CHCl_2CClF_2$, $CClF=CCl_2$, and $CHCl_2CCl_2F$, can be recycled to the reactor for the production of additional FC-123 and FC-124. In addition, FC-123 can be recycled to the reactor for the production of additional FC-124 when this is desired.

The reaction of the tetrahaloethylene with HF may be conducted in any suitable reactor, including fixed and fluidized bed reactors. The reaction vessel should be constructed from materials which are resistant to the corrosive effects of hydrogen fluoride such as Hastelloy and Inconel.

Pressure is not critical. Atmospheric and superatmospheric pressures are the most convenient and are therefore preferred.

The fluorinated alkanes produced by the invention have utility as blowing agents and refrigerants. They can also be used as starting materials for the preparation of other useful compounds. For example, FC-124 can be used to prepare 1,1,1,2-tetrafluoroethane.

## EXAMPLES

In the following illustrative examples, all parts and percentages are by weight and all temperatures are Centigrade unless otherwise stated. All reactions used commercial HF containing only trace amounts of water.

### General Procedure for Fluorination

The reactor (a (0.5 inch ID, 12 inch long) 0.3m long Inconel pipe) was charged with the amount of unfluorinated catalyst as described in the following examples, and placed in a sand bath. The bath was gradually heated to 400° while nitrogen gas at a flow rate of 50 ml/minute was passed through the reactor to remove traces of water. The temperature was lowered to 200° and HF and nitrogen gas (1:4 molar ratio) were passed through the reactor and the nitrogen flow was decreased with time until neat HF was being passed through the reactor. At this point, the temperature was gradually raised to 450° and maintained there for 15 to 300 minutes. The fluorine content of the catalyst composition corresponded to an $AlF_3$ content, exclusive of the metal, of at least 95%.

The initial fluorine content of the catalyst composition can be determined to correspond to an $AlF_3$content, exclusive of the metal, of at least 90%. This determination is based on the following calculation related to this reaction:

$$Al_2O_3 + 6\ HF \dashrightarrow 2\ AlF_3 + 3\ H_2O$$

y = weight of unfluorinated catalyst composition which has been dried at a temperature of at least

400°C for at least four hours in a stream of dry nitrogen, air or other suitable inert medium, minus the weight of metal compound which is in the unfluorinated catalyst composition.

z = weight of fluorinated catalyst composition minus the weight of metal compound calculated as metal fluoride.

Let x = weight of $Al_2O_3$ remaining after fluorination
y - x = weight of reacted $Al_2O_3$
z - x = weight of $AlF_3$ in the fluorinated alumina

$$(y - x)168/102 = z - x$$

Weight of $AlF_3$ as % of dry fluorinated alumina can then be calculated as follows:

$$\frac{(z - x)}{z} 100\%$$

The temperature was then decreased to the indicated values and, thereafter, $CCl_2=CCl_2$ flow was started. The flows of HF and $CCl_2=CCl_2$ were adjusted to give the indicated molar ratios and contact times. diameter, column containing "Krytox" perfluorinated polyether on an inert support and a helium flow of 35 cc/minute. Gas chromatographic conditions were 70° for 3 minutes followed by temperature programming to 180° at a rate of 6°/minute.

EXAMPLES 1 - 2

The General Procedure for Fluorination was followed using 19.8 g. (30 cc) of $NiCl_2/Al_2O_3$ (2% Ni) as the initial catalyst charge. The results of the reaction of HF with $CCl_2=CCl_2$ over the prepared catalyst are given in Table 1.

TABLE 1

| | Example | |
|---|---|---|
| | 1 | 2 |
| Temp. °C | 325 | 350 |
| $HF/C_2Cl_4$ (mol ratio) | 5/1 | 6/1 |
| Contact Time (sec.) | 45 | 30 |
| Conversion % | 58.7 | 61.2 |
| | Area Percent | |
| $CF_3CHCl_2$ | 70.0 | 60.6 |
| $CF_3CHClF$ | 12.4 | 20.3 |
| $CF_3CHF_2$ | 0.5 | 1.3 |
| $CF_3CHCl_2$ plus $CF_3CHClF$ | 82.4 | 80.9 |

The reactor effluent was scrubbed with aqueous potassium hydroxide to remove HCl and HF and sampled on-line with a Hewlett Packard HP 5890 gas chromatograph using a (6m long 3mm diameter 20 foot long, one-eighth inch diameter), column containing "Krytox" perfluorinated polyether on an inert support and a helium flow of 35 cc/minute. Gas chromatographic conditions were 70° for 3 minutes followed by temperature programming to 180° at a rate of 6°/minute.

EXAMPLES 3 - 4

The General Procedure for Fluorination was followed using 19.5 g. (30 cc) of $MnCl_2/Al_2O_3$ (1.87% Mn) as the initial catalyst charge. The results of the reaction of HF with $CCl_2=CCl_2$ over the prepared catalyst are given in Table 2.

TABLE 2

| | Example | |
|---|---|---|
| | 3 | 4 |
| Temp. °C HF/$C_2Cl_4$ | 350 | 375 |
| (mol ratio) | 6/1 | 10/1 |
| Contact Time (sec.) | 30 | 25 |
| Conversion % | 70.4 | 61.5 |
| | Area Percent | |
| $CF_3CHCl_2$ | 67.8 | 58.5 |
| $CF_3CHClF$ | 18.5 | 17.6 |
| $CF_3CHF_2$ | 1.0 | 1.1 |
| $CF_3CHCl_2$ plus $CF_3CHClF$ | 86.3 | 76.1 |

EXAMPLES 5 - 9

The General Procedure for Fluorination was followed using 18.4 g. (30 cc) of $CoCl_2/Al_2O_3$ (2.0% Co) as the initial catalyst charge. The results of the reaction of HF with $CCl_2=CCl_2$ over the prepared catalyst are given in Table 3.

TABLE 3

| | Example | | | | |
|---|---|---|---|---|---|
| | 5 | 6 | 7 | 8 | 9 |
| Temp. °C | 350 | 350 | 350 | 350 | 350 |
| HF/$C_2Cl_4$ (mol ratio) | 7/1 | 10/1 | 5/1 | 5/1 | 3/1 |
| Contact Time (sec.) | 13.2 | 19.3 | 31.8 | 17.6 | 25.7 |
| Conversion % | 87.3 | 90.3 | 86.8 | 75.7 | 60.0 |
| | Area Percent | | | | |
| $CF_3CHCl_2$ | 56.5 | 52.8 | 53.6 | 57.8 | 52.1 |
| $CF_3CHClF$ | 37.2 | 41.1 | 39.4 | 32.2 | 32.9 |
| $CF_3CHF_2$ | 1.6 | 2.1 | 2.2 | 1.5 | 2.5 |
| $CF_3CHCl_2$ plus $CF_3CHClF$ | 93.7 | 93.9 | 93.0 | 90.0 | 85.0 |

EXAMPLES 10 - 13

The General Procedure for Fluorination was followed using 20.4 g. (30 cc) of $CrCl_3/Al_2O_3$ (5.2% Cr) as the initial catalyst charge. The results of the reaction of HF with $CCl_2=CCl_2$ over the prepared catalyst are given in Table 4.

TABLE 4

| | Example | | | |
|---|---|---|---|---|
| | 10 | 11 | 12 | 13 |
| Temp. °C | 425 | 350 | 350 | 350 |
| $HF/C_2Cl_4$ (mol ratio) | 4/1 | 4/1 | 5/1 | 6/1 |
| Contact Time (sec.) | 15 | 60 | 60 | 60 |
| Conversion % | 62.1 | 78.3 | 78.1 | 80.3 |
| | Area Percent | | | |
| $CF_3CHCl_2$ | 31.4 | 50.8 | 49.6 | 46.1 |
| $CF_3CHClF$ | 22.2 | 28.5 | 29.7 | 33.3 |
| $CF_3CHF_2$ | 9.8 | 6.9 | 7.3 | 7.2 |
| $CF_3CHCl_2$ plus $CF_3CHClF$ | 53.6 | 79.3 | 79.3 | 79.4 |

EXAMPLES 14 - 19

The general Procedure for Fluorination was followed using 20.4 g. (30 cc) of $CoCl_2/Al_2O_3$ (2% Co) as the initial catalyst charge. The ratio of $HF/C_2Cl_4$ was 6/1 and the contact time was 30 seconds. The results of the reaction of HF with $CCl_2=CCl_2$ over the prepared catalyst are given in Table 5.

TABLE 5

| | Example | | | | | |
|---|---|---|---|---|---|---|
| | 14 | 15 | 16 | 17 | 18 | 19 |
| Temp. °C | 265 | 255 | 245 | 235 | 225 | 215 |
| Conversion % | 36.2 | 29.9 | 25.3 | 21.3 | 16.5 | 12.9 |
| | Area Percent | | | | | |
| $CF_3CHCl_2$ | 54.1 | 43.1 | 32.8 | 23.4 | 11.3 | 4.7 |
| $CF_3CHClF$ | 1.1 | 1.0 | 0.4 | 0.0 | 0.0 | 0.0 |
| $CClF_2CHCl_2$ | 29.6 | 40.6 | 50.2 | 58.7 | 52.1 | 43.7 |
| $CCl_2FCHCl_2$ | 0.6 | 0.7 | 1.2 | 1.9 | 1.9 | 2.3 |
| Other | 14.6 | 14.6 | 15.4 | 16.0 | 34.7 | 49.3 |

**Claims**

**Claims for the following Contracting States : AT, BE, IT, LU**

**1.** A process for the preparation of 1,1,1-trifluorodichloroethane and/or 1,1,1,2-tetrafluorochloroethane by fluorination of a tetrahaloethylene, $C_2Cl_{4-x}F_x$, wherein x = 0 to 3, comprising contacting in the gaseous phase at 275 °C to 450 °C said tetrahaloethylene and HF with a catalyst comprising at least one metal in an oxidation state greater than zero, said metal selected from the group consisting of chromium, manganese, rhodium, nickel, cobalt and mixtures thereof, said metal in combination with a high fluorine content aluminium-containing compound consisting essentially of aluminium and fluorine in such proportions that the fluorine content corresponds to an $AlF_3$ content of at least 90% by weight of the catalyst exclusive of said metal, said $AlF_3$ content being obtained by pretreatment of the unfluorinated catalyst with a vaporizable fluorine-containing compound, and

other than a process comprising contacting in the gaseous phase at 300 °C to 460 °C with a HF in the presence of a catalyst comprising at least one metal selected from chromium, manganese, nickel and cobalt on a support comprising aluminium oxygen and fluorine in such proportions that the fluorine content corresponds to an $AlF_3$ content of at least 90% by weight of the catalyst composition exclusive of the metal said $AlF_3$ content being obtained by pretreatment.

2. The process of Claim 1 wherein the tetrahaloethylene is tetrachloroethylene.

3. The process of Claim 1 wherein the catalyst contains 0.02 to 20 weight percent of said metal expressed as the divalent oxide.

4. The process of Claim 1 wherein the catalyst contains 0.1 to 5 weight percent of said metal expressed as the divalent oxide.

5. The process as in Claims 1, 2 or 3 wherein the HF is contacted with the tetrahaloethylene at a mol ratio of 1/1 to 20/1, at a temperature of 275 °C, and a contact time of 5 to 100 seconds.

6. The process of claim 4 wherein conversion of the tetrahaloethylene to fluorinated products is between 30% and 95%.

7. The process of Claim 1 wherein the said metal is selected from the group consisting of manganese, rhodium, nickel and cobalt.

8. The process of Claim 6 wherein the said metal is cobalt.

9. The process of Claim 1 further comprising the step of recycling at least a portion of the $CF_3CHCl_2$ produced to the contacting step of conversion to additional $CF_3CHClF$.

10. The process of Claim 1 wherein the vaporizable fluorine-containing compound is selected from the group consisting of HF, $SF_4$, $CCl_3F$, $CCl_2F_2$, $CHF_3$ or $CCl_2FCCl_3F_2$.

**Claims for the following Contracting Sates : CH, LI, DE, FR, GB, NL, SE**

1. A process for the preparation of 1,1,1,-trifluorodichloroethane and/or 1,1,1,2-tetrafluorochloroethane by fluorination of a tetrahaloethylene, $C_2Cl_{4-x}F_x$, wherein x = 0 to 3, comprising contacting in the gaseous phase at 275 °C to 450 °C said tetrahaloethylene and HF with a catalyst comprising at least one metal in an oxidation state greater than zero, said metal selected from the group consisting of chromium, manganese, rhodium, nickel, cobalt and mixtures thereof, said metal in combination with a high fluorine content aluminium-containing compound consisting essentially of aluminium and fluorine in such proportions that the fluorine content corresponds to an $AlF_3$ content of at least 90% by weight of the catalyst exclusive of said metal, said $AlF_3$ content being obtained by pretreatment of the unfluorinated catalyst with a vaporizable fluorine-containing compound, other than a process for the preparation of 1,1,1-trifluoro-2,2-dichloroethane which comprises reacting perchloroethylene with hydrogen fluoride in the gas phase in the presence of a catalyst comprising 1 to 15 weight % $Cr_20_3$ calculated as Cr supported on $AlF_3$ in the gamma and/or beta form, and
   other than a process comprising contacting in the gaseous phase at 300 °C to 460 °C with a HF in the presence of a catalyst comprising at least one metal selected from chromium manganese nickel and cobalt on a support comprising aluminum oxygen and fluorine in such proportions that the fluorine content corresponds to an $AlF_3$ content of at least 90% by weight of the catalyst composition exclusive of the metal said $AlF_3$ content being obtained by pretreatment.

2. The process of Claim 1 wherein the tetrahaloethylene is tetrachloroethylene.

3. The process of Claim 1 wherein the catalyst contains 0.02 to 20 weight percent of said metal expressed as the divalent oxide.

4. The process of Claim 1 wherein the catalyst contains 0.1 to 5 weight percent of said metal expressed as the divalent oxide.

5. The process as in Claims 1, 2 or 3 wherein the HF is contacted with the tetrahaloethylene at a mol ratio of 1/1 to 20/1, at a temperature of 275 °C, and a contact time of 5 to 100 seconds.

6. The process of claim 4 wherein conversion of the tetrahaloethylene to fluorinated products is between 30% and 95%.

7. The process of Claim 1 wherein the said metal is selected from the group consisting of manganese, rhodium, nickel and cobalt.

8. The process of Claim 6 wherein the said metal is cobalt.

9. The process of Claim 1 further comprising the step of recycling at least a portion of the $CF_3CHCl_2$ produced to the contacting step of conversion to additional $CF_3CHClF$.

10. The process of Claim 1 wherein the vaporizable fluorine-containing compound is selected from the group consisting of HF, $SF_4$, $CCl_3F$, $CCl_2F_2$, $CHF_3$ or $CCl_2FCCl_3F_2$.

**Patentansprüche**
**Patentansprüche für folgende Vertragsstaaten : AT, BE, IT, LU**

1. Verfahren zur Herstellung von 1,1,1-Trifluordichlorethan und/oder 1,1,1,2-Tetrafluorchlorethan durch Fluorierung eines Tetrahalogenethylens $C_2Cl_{4-x}F_x$, worin x = 0 bis 3, umfassend Zusammenbringen in der Gasphase bei 275 °C bis 450 °C des genannten Tetrahalogenethylens und von HF mit einem Katalysator, umfassend wenigstens ein Metall in einer Oxidationsstufe größer als 0, wobei das genannte Metall ausgewählt wird aus der Gruppe, bestehend aus Chrom, Mangan, Rhodium, Nickel, Cobalt und Gemischen davon, das genannte Metall in Kombination mit einer aluminiumhaltigen Verbindung mit einem hohen Fluorgehalt, bestehend im wesentlichen aus Aluminium und Fluor in solchen Anteilen, daß der Fluorgehalt einem $AlF_3$-Gehalt von wenigstens 90 Gew.-% des Katalysators ausschließlich des genannten Metalls entspricht, wobei der genannte $AlF_3$-Gehalt erhalten wird durch Vorbehandeln des unfluorierten Katalysators mit einer verdampfbaren fluorhaltigen Verbindung, und anders als ein Verfahren, das das Zusammenbringen in der Gasphase bei 300 °C bis 460 °C mit HF in Gegenwart eines Katalysators umfaßt, der wenigstens ein Metall umfaßt, ausgewählt aus Chrom, Mangan, Nickel und Cobalt auf einem Träger, umfassend Aluminiumsauerstoff und Fluor in solchen Anteilen, daß der Fluorgehalt einem $AlF_3$-Gehalt von wenigstens 90 Gew.-% der Katalysatorzusammensetzung ausschließlich des Metalls entspricht, wobei der genannte $AlF_3$-Gehalt durch Vorbehandeln erhalten wird.

2. Verfahren nach Anspruch 1, bei dem das Tetrahalogenethylen Tetrachlorethylen ist.

3. Verfahren nach Anspruch 1, bei dem der Katalysator 0,02 bis 20 Gew.-% des genannten Metalls, ausgedrückt als zweiwertiges Oxid, enthält.

4. Verfahren nach Anspruch 1, bei dem der Katalysator 0,1 bis 5 Gew.-% des genannten Metalls, ausgedrückt als zweiwertiges Oxid, enthält.

5. Verfahren nach den Ansprüchen 1, 2 oder 3, bei dem HF mit dem Tetrahalogenethylen in einem Molverhältnis von 1/1 bis 20/1 bei einer Temperatur von 275 °C und einer Kontaktzeit von 5 bis 100 Sekunden in Kontakt gebracht wird.

6. Verfahren nach Anspruch 4, bei dem die Umwandlung des Tetrahalogenethylens zu fluorierten Produkten zwischen 30 % und 95 % liegt.

7. Verfahren nach Anspruch 1, bei dem das genannte Metall ausgewählt wird aus der Gruppe, bestehend aus Mangan, Rhodium, Nickel und Cobalt.

8. Verfahren nach Anspruch 6, bei dem das genannte Metall Cobalt ist.

9. Verfahren nach Anspruch 1, das außerdem die Stufe der Wiederverwendung in der Kontaktstufe der Umwandlung zu zusätzlichem $CF_3CHClF$ von wenigstens einem Teil des erzeugten $CF_3CHCl_2$ umfaßt.

10. Verfahren nach Anspruch 1, bei dem die verdampfbare fluorhaltige Verbindung ausgewählt wird aus der Gruppe, bestehend aus HF, $SF_4$, $CCl_3F$, $CCl_2F_2$, $CHF_3$ oder $CCl_2FCCl_3F_2$.

**Patentansprüche für folgende Vertragsstaaten : CH, LI, DE, FR, GB, NL, SE**

1. Verfahren zur Herstellung von 1,1,1-Trifluordichlorethan und/oder 1,1,1,2-Tetrafluorchlorethan durch Fluorierung eines Tetrahalogenethylens $C_2Cl_{4-x}F_x$, worin x = 0 bis 3, umfassend Zusammenbringen in der Gasphase bei 275 °C bis 450 °C des genannten Tetrahalogenethylens und von HF mit einem Katalysator, umfassend wenigstens ein Metall in einer Oxidationsstufe größer als 0, wobei das genannte Metall ausgewählt wird aus der Gruppe, bestehend aus Chrom, Mangan, Rhodium, Nickel, Cobalt und Gemischen davon, das genannte Metall in Kombination mit einer aluminiumhaltigen Verbindung mit einem hohen Fluorgehalt, bestehend im wesentlichen aus Aluminium und Fluor in solchen Anteilen, daß der Fluorgehalt einem $AlF_3$-Gehalt von wenigstens 90 Gew.-% des Katalysators ausschließlich des genannten Metalls entspricht, wobei der genannte $AlF_3$-Gehalt erhalten wird durch Vorbehandeln des unfluorierten Katalysators mit einer verdampfbaren fluorhaltigen Verbindung, und anders als ein Verfahren zur Herstellung von 1,1,1-Trifluor-2,2-dichlorethan, das die Umsetzung von Perchlorethylen mit Fluorwasserstoff in der Gasphase in Gegenwart eines Katalysators umfaßt, der 1 bis 15 Gew.-% $Cr_2O_3$, berechnet als Cr, trägergebunden auf $AlF_3$, in der $\gamma$- und/oder $\beta$-Form umfaßt, und anders als ein Verfahren, das das Zusammenbringen in der Gasphase bei 300 °C bis 460 °C mit HF in Gegenwart eines Katalysators umfaßt, der wenigstens ein Metall umfaßt, ausgewählt aus Chrom, Mangan, Nickel und Cobalt auf einem Träger, umfassend Aluminiumsauerstoff und Fluor in solchen Anteilen, daß der Fluorgehalt einem $AlF_3$-Gehalt von wenigstens 90 Gew.-% der Katalysatorzusammensetzung, ausschließlich des Metalls, entspricht, wobei der genannte $AlF_3$-Gehalt durch Vorbehandeln erhalten wird.

2. Verfahren nach Anspruch 1, bei dem das Tetrahalogenethylen Tetrachlorethylen ist.

3. Verfahren nach Anspruch 1, bei dem der Katalysator 0,02 bis 20 Gew.-% des genannten Metalls, ausgedrückt als zweiwertiges Oxid, enthält.

4. Verfahren nach Anspruch 1, bei dem der Katalysator 0,1 bis 5 Gew.-% des genannten Metalls, ausgedrückt als zweiwertiges Oxid, enthält.

5. Verfahren nach den Ansprüchen 1, 2 oder 3, bei dem HF mit dem Tetrahalogenethylen in einem Molverhältnis von 1/1 bis 20/1 bei einer Temperatur von 275 °C und einer Kontaktzeit von 5 bis 100 Sekunden in Kontakt gebracht wird.

6. Verfahren nach Anspruch 4, bei dem die Umwandlung des Tetrahalogenethylens zu fluorierten Produkten zwischen 30 % und 95 % liegt.

7. Verfahren nach Anspruch 1, bei dem das genannte Metall ausgewählt wird aus der Gruppe, bestehend aus Mangan, Rhodium, Nickel und Cobalt.

8. Verfahren nach Anspruch 6, bei dem das genannte Metall Cobalt ist.

9. Verfahren nach Anspruch 1, das außerdem die Stufe der Wiederverwendung in der Kontaktstufe der Umwandlung zu zusätzlichem $CF_3CHClF$ von wenigstens einem Teil des erzeugten $CF_3CHCl_2$ umfaßt.

10. Verfahren nach Anspruch 1, bei dem die verdampfbare fluorhaltige Verbindung ausgewählt wird aus der Gruppe, bestehend aus HF, $SF_4$, $CCl_3F$, $CCl_2F_2$, $CHF_3$ oder $CCl_2FCCl_3F_2$.

**Revendications**

**Revendications pour les Etats contractants suivants : AT, BE, IT, LU**

1. Un procédé pour la préparation de 1,1,1-trifluorodichloroéthane et/ou de 1,1,1,2-tétrafluorochloroéthane par fluoration d'un tétrahalogénoéthylène, $C_2Cl_{4-x}F_x$, dans lequel x = 0 à 3, consistant à mettre en contact en phase gazeuse à une température comprise entre 275° C et 450° C ledit tétrahalogénoéthylène et du HF avec un catalyseur comprenant au moins un métal dans un état d'oxydation supérieur à zéro, ledit métal étant choisi parmi le groupe composé du chrome, du manganèse, du rhodium, du nickel, du cobalt et des mélanges de ceux-ci, ledit métal étant en combinaison avec un composé contenant de l'aluminium à forte teneur en fluor consistant essentiellement en de l'aluminium et du

fluor dans des proportions telles que la teneur en fluor corresponde à une teneur en $AlF_3$ d'au moins 90 pour cent en poids du catalyseur à l'exclusion dudit métal, ladite teneur en $AlF_3$ étant obtenue par prétraitement du catalyseur non fluoré avec un composé vaporisable contenant du fluor, et

autre qu'un procédé comprenant la mise en contact en phase gazeuse à une température comprise entre 300° C et 460° C avec du HF en présence d'un catalyseur comprenant au moins un métal choisi parmi le chrome, le manganèse, le nickel et le cobalt sur un support comprenant de l'aluminium, de l'oxygène et du fluor dans des proportions telles que la teneur en fluor correspond à une teneur en $AlF_3$ d'au moins 90 pour cent en poids de la composition du catalyseur à l'exclusion du métal, ladite teneur en $AlF_3$ étant obtenue par prétraitement.

**2.** Le procédé de la revendication 1 dans lequel le tétrahalogénoéthylène est le tétrachloroéthylène.

**3.** Le procédé de la revendication 1 dans lequel le catalyseur contient 0,02 à 20 pour cent en poids dudit métal exprimé sous forme de l'oxyde divalent.

**4.** Le procédé de la revendication 1 dans lequel le catalyseur contient 0,1 à 5 pour cent en poids dudit métal exprimé sous forme de l'oxyde divalent.

**5.** Le procédé de la revendication 1, 2 ou 3 dans lequel le HF est mis en contact avec le tétrahalogénoéthylène à un rapport molaire de 1/1 à 20/1, à une température de 275° C, et pendant une durée de contact de 5 à 100 secondes.

**6.** Le procédé de la revendication 4 dans lequel la conversion du tétrahalogénoéthylène en produits fluorés est entre 30 % et 95 %.

**7.** Le procédé de la revendication 1 dans lequel ledit métal est choisi parmi le groupe composé du manganèse, du rhodium, du nickel et du cobalt.

**8.** Le procédé de la revendication 6 dans lequel ledit métal est du cobalt.

**9.** Le procédé de la revendication 1 comprenant en outre l'étape de recyclage d'au moins une partie du $CF_3CHCl_2$ produit à l'étape de mise en contact pour conversion en $CF_3CHClF$ supplémentaire.

**10.** Le procédé de la revendication 1 dans lequel le composé vaporisable contenant du fluor est choisi parmi le groupe composé de HF, $SF_4$, $CCl_3F$, $CCl_2F_2$, $CHF_3$ ou $CCl_2FCCl_3F_2$.

**Revendications pour les Etats contractants suivants: CH, LI, DE, FR, GB, NL, SE**

**1.** Un procédé pour la préparation de 1,1,1-trifluorodichloroéthane et/ou de 1,1,1,2-tétrafluorochloroéthane par fluoration d'un tétrahalogénoéthylène, $C_2Cl_{4-x}F_x$, dans lequel x = 0 à 3, consistant à mettre en contact en phase gazeuse à une température comprise entre 275° C et 450° C ledit tétrahalogénoéthylène et du HF avec un catalyseur comprenant au moins un métal dans un état d'oxydation supérieur à zéro, ledit métal étant choisi parmi le groupe composé du chrome, du manganèse, du rhodium, du nickel, du cobalt et des mélanges de ceux-ci, ledit métal étant en combinaison avec un composé contenant de l'aluminium à forte teneur en fluor consistant essentiellement en de l'aluminium et du fluor dans des proportions telles que la teneur en fluor corresponde à une teneur en $AlF_3$ d'au moins 90 pour cent en poids du catalyseur à l'exclusion dudit métal, ladite teneur en $AlF_3$ étant obtenue par prétraitement du catalyseur non fluoré avec un composé vaporisable contenant du fluor, autre qu'un procédé pour la préparation de 1,1,1-trifluoro-2,2-dichloroéthane qui comprend la réaction du perchloroéthylène avec du fluorure d'hydrogène en phase gazeuse en présence d'un catalyseur comprenant 1 à 15 pour cent en poids de $Cr_2O_3$ calculé comme Cr supporté sur $AlF_3$ sous la forme gamme et/ou bêta, et

autre qu'un procédé comprenant la mise en contact en phase gazeuse à une température comprise entre 300° C et 460° C avec du HF en présence d'un catalyseur comprenant au moins un métal choisi parmi le chrome, le manganèse, le nickel et le cobalt sur un support comprenant de l'aluminium, de l'oxygène et du fluor dans des proportions telles que la teneur en fluor correspond à une teneur en $AlF_3$ d'au moins 90 pour cent en poids de la composition du catalyseur à l'exclusion du métal, ladite teneur en $AlF_3$ étant obtenue par prétraitement.

2. Le procédé de la revendication 1 dans lequel le tétrahalogénoéthylène est le tétrachloroéthylène.

3. Le procédé de la revendication 1 dans lequel le catalyseur contient 0,02 à 20 pour cent en poids dudit métal exprimé sous forme de l'oxyde divalent.

4. Le procédé de la revendication 1 dans lequel le catalyseur contient 0,1 à 5 pour cent en poids dudit métal exprimé sous forme de l'oxyde divalent.

5. Le procédé de la revendication 1, 2 ou 3 dans lequel le HF est mis en contact avec le tétrahalogénoé-thyléne à un rapport molaire de 1/1 à 20/1, à une température de 275° C, et pendant une durée de contact de 5 à 100 secondes.

6. Le procédé de la revendication 4 dans lequel la conversion du tétrahalogénoéthylène en produits fluorés est entre 30 % et 95 %.

7. Le procédé de la revendication 1 dans lequel ledit métal est choisi parmi le groupe composé du manganèse, du rhodium, du nickel et du cobalt.

8. Le procédé de la revendication 6 dans lequel ledit métal est du cobalt.

9. Le procédé de la revendication 1 comprenant en outre l'étape de recyclage d'au moins une partie du $CF_3CHCl_2$ produit à l'étape de mise en contact pour conversion en $CF_3CHClF$ supplémentaire.

10. Le procédé de la revendication 1 dans lequel le composé vaporisable contenant du fluor est choisi parmi le groupe composé de HF, $SF_4$, $CCl_3F$, $CCl_2F_2$, $CHF_3$ ou $CCl_2FCCl_3F_2$.